# EUROPEAN PATENT APPLICATION

(11) **EP 4 570 248 A1**
(43) Date of publication of application: **18.06.2025**
(21) Application number: 23852589.3
(22) Date of filing: 09.08.2023
(51) Int. Cl.: A61K 31/436, A61K 9/06, A61K 9/08, A61K 9/10, A61K 9/70, A61P 17/00, A61P 29/00, C12N 15/12, C12N 15/28

(54) **SIROLIMUS-CONTAINING TOPICAL DRUG FOR TREATING SKIN HARDENING IN SYSTEMIC SCLERODERMA**

(30) Priority: 12.08.2022 JP 2022129124
(71) Applicant: National University Corporation Gunma University, Maebashi-shi, Gunma 371-8510 (JP)
(72) Inventor: MOTEGI, Sei-ichiro, Maebashi-shi, Gunma 371-8510 (JP)
(74) Representative: Gill Jennings & Every LLP
(86) International application number: PCT/JP2023/029015
(87) International publication number: WO 2024/034627

(57) **Abstract**

A novel medicine for treating or preventing skin hardening in systemic sclerosis was discovered due to the discovery that skin hardening can be ameliorated by administering a skin external medicine including sirolimus to the skin in systemic sclerosis.

## Description

### Technical Field

The present invention relates to a skin external medicine for treating skin hardening in systemic sclerosis, the skin external medicine including sirolimus.

### Background Art

Systemic sclerosis is a disease in which an extracellular matrix such as collagen accumulates in the systemic skin or organ, whereby the fibrosis of the skin or the organ is caused to sclerose the skin or the organ. Systemic sclerosis is a specified rare and intractable disease. There is no definitive treatment for systemic sclerosis. When systemic sclerosis occurs, flexion contracture or a limited range of joint motion, caused by skin hardening, leads to significantly diminished quality of life. There is no evidence-based useful treatment against skin hardening, and many patients orally take a large amount of adrenocortical hormone (steroid). Therefore, there are problems in that various side effects occur. Therefore, an effective treatment method with a few side effects has been earnestly desired.

It is disclosed that symptoms associated with systemic sclerosis, such as skin fibrosis, were ameliorated by oral administration of sirolimus (also known as rapamycin) to patients with systemic sclerosis (Non Patent Literature 1). Moreover, it is disclosed that intraperitoneal injection of sirolimus (hereinafter also referred to as rapamycin) allowed skin fibrosis in scleroderma model mice (bleomycin-induced scleroderma model mice and tight skin mice) to be suppressed, that is, the growth of fibroblasts and the production of collagen to be dose-dependently suppressed (Non Patent Literature 2).

A sirolimus-containing gel (Nobelpharma Co., Ltd., product name: RAPALIMUS (registered trademark) Gel 0.2%, 2 mg of sirolimus in 1 g) is an external medicine indicated for skin lesions, such as angiofibroma, associated with nodular sclerosis. Nodular sclerosis is a disease in which an mTOR (mechanistic target of rapamycin) signal is constantly activated by gene mutation. External use of the sirolimus-containing gel for skin lesions, such as angiofibroma, associated with nodular sclerosis is known to cause suppression of the mTOR signal to exert a therapeutic effect.

Any clinically usable sirolimus external medicine other than the external medicine indicated for skin lesions, such as angiofibroma, associated with nodular sclerosis has not existed. Thus, there has been no report on examination of the effect of ameliorating skin hardening in systemic sclerosis by using sirolimus as a skin external medicine, and there has been no report on a therapeutic effect in the case of applying a skin external medicine containing sirolimus for skin hardening in systemic sclerosis.

### Citation List

### Non Patent Literature

Non Patent Literature 1: Arthritis Rheum., 2009; 60: 3821-3830
Non Patent Literature 2: Arthritis Rheum., 2010: 62: 2476-2487
Non Patent Literature 3: Arthritis Rheum., 2000; 43: 2455-2463
Non Patent Literature 4: Arthritis Rheum., 2004; 50: 1918-1927
Non Patent Literature 5: Ann Rheum Dis., 2005; 64: 1233-1235
Non Patent Literature 6: N Engl J Med., 2006; 354: 2655-2666
Non Patent Literature 7: J Transplant., 2009; 2009: 701464

### Summary of Invention

### Technical Problem

The present invention addresses an objective of providing a medicine for treating or preventing skin hardening in systemic sclerosis.

### Solution to Problem

As a result of intensive examination for solving the problem described above, it was found that the significant effect of ameliorating skin hardening is obtained by applying a skin external medicine containing sirolimus to a scleroderma model mouse (hereinafter also referred to as "scleroderma-like skin hardening model mouse") produced by subcutaneous administration of bleomycin. The present invention was thus accomplished.

In other words, the present invention is as follows.
[1] A skin external medicine for treatment or prevention of skin hardening in systemic sclerosis, wherein the skin external medicine comprises sirolimus.
[2] The skin external medicine according to [1], wherein the treatment or the prevention results in suppression of early-stage skin fibrosis.
[3] The skin external medicine according to [1] or [2], wherein a dosage form is a gel, a cream, an ointment, a liquid, a lotion, or a patch.
[4] The skin external medicine according to any of [1] to [3], wherein the skin external medicine comprises sirolimus at a rate of 0.2% by mass or more with respect to a total of the skin external medicine.
[5] The skin external medicine according to any of [1] to [4], wherein production of collagen is suppressed in a skin to which the skin external medicine is administered.
[6] The skin external medicine according to any of [1] to [5], wherein inflammation is suppressed in a skin to which the skin external medicine is administered.
[7] A method of treating or preventing skin hardening in systemic sclerosis, wherein the method comprises administering sirolimus to a skin of a subject in need thereof.
[8] Sirolimus for use in treatment or prevention of skin hardening in systemic sclerosis by administration of the sirolimus to a skin.
[9] Use of sirolimus in manufacturing of a skin external medicine for treating or preventing skin hardening in systemic sclerosis.

### Advantageous Effects of Invention

Skin hardening can be ameliorated by applying a skin external medicine containing sirolimus to the skin with systemic sclerosis. Therefore, skin hardening in systemic sclerosis for which any effective treatment method does not currently exist can be treated or prevented by a method having a few systemic side effects.

### Brief Description of Drawings

Figure 1 is a diagram illustrating a schedule of administration of sirolimus gel to the skins with early-stage fibrosis in scleroderma-like skin hardening model mice.
Figure 2 is a microphotograph illustrating the suppressive effect of sirolimus gel on skin fibrosis in the scleroderma-like skin hardening model mice. In the figure, portions indicated by the arrows represent dermal thicknesses.
Figure 3 is a diagram illustrating the suppressive effect of the sirolimus gel on the skin fibrosis in the scleroderma-like skin hardening model mice. The result of a measured dermal thickness in each mouse is illustrated.
Figure 4 is a diagram illustrating the suppressive effect of sirolimus gel on skin fibrosis in scleroderma-like skin hardening model mice. The result of the measured amount of soluble collagen in the skin tissue of each mouse is illustrated.
Figure 5 is a diagram illustrating a schedule of administration of sirolimus gel to the fibrotic skins in scleroderma-like skin hardening model mice.
Figure 6 is a diagram illustrating the therapeutic effect of sirolimus gel on skin fibrosis in scleroderma-like skin hardening model mice. The result of the measured amount of soluble collagen in the skin tissue of each mouse is illustrated.
Figure 7 is a microphotograph illustrating the effect of sirolimus gel on the inflammation of skin fibrosis sites in scleroderma-like skin hardening model mice. CD3 positive cells and CD68 positive cells in the skin tissue of each mouse were stained. The length of the scale bar is 50 µm.
Figure 8 is a diagram illustrating the effect of sirolimus gel on the inflammation of skin fibrosis sites in scleroderma-like skin hardening model mice. The result of the measured number of CD3 positive cells in the skin tissue of each mouse is illustrated.
Figure 9 is a diagram illustrating the effect of sirolimus gel on the inflammation of skin fibrosis sites in scleroderma-like skin hardening model mice. The result of the measured number of CD68 positive cells in the skin tissue of each mouse is illustrated.
Figure 10 is a diagram illustrating the effect of sirolimus gel on the inflammation of skin fibrosis sites in scleroderma-like skin hardening model mice. The result of the measured expression level of TNF-α in the skin tissue of each mouse is illustrated.
Figure 11 is a diagram illustrating the effect of sirolimus gel on the inflammation of skin fibrosis sites in scleroderma-like skin hardening model mice. The result of the measured expression level of TGF-β in the skin tissue of each mouse is illustrated.
Figure 12 is a microphotograph illustrating the effect of sirolimus gel on the expression of phosphorylated mTOR in skin fibrosis sites in scleroderma-like skin hardening model mice. Phosphorylated mTOR expressed cells in the skin tissue of each mouse were stained.
Figure 13 is a diagram illustrating the effect of sirolimus gel on the expression of phosphorylated mTOR in skin fibrosis sites in scleroderma-like skin hardening model mice. The result of the measured number of CD68 positive cells in the skin tissue of each mouse is illustrated.
Figure 14 is a photograph illustrating the effect of sirolimus gel on the expression of phosphorylated mTOR in skin fibrosis sites in scleroderma-like skin hardening model mice. The result of the confirmation of the expression level of phosphorylated mTOR by carrying out Western blotting method for a sample derived from the skin tissue of each mouse is illustrated.

### Description of Embodiments

One embodiment of the present invention is a skin external medicine for treating and/or preventing skin hardening in systemic sclerosis, wherein the skin external medicine contains sirolimus.

Sirolimus is a macrolide compound. Sirolimus can be represented by, for example, IUPAC name:
(3S,6R,7E,9R,1OR,12R,14S,15E,17E,19E,21S,23S,26R,27R,34aS)-9,10,12, 13, 14,21,22,23,24,25,26,27,32,33,34,34a-hexadecahydro-9,27-dihydroxy-3-[(1R)-2-[(1S,3R,4R)-4-hydroxy-3-methoxycyclohexyl]-1-methylethyl]-10,21-dimethoxy-6,8,12,14,20,26-hexamethyl-23,27-epoxy-3H-pyrido[2,1-c][1,4]-oxazacyclohentriacontine-1,5,11,28,29(4H,6H,3 1H)-pentone, and can be represented by CAS number 53123-88-9.

Unless the effects of the present invention are adversely influenced, sirolimus may be a derivative thereof, or the functional group of sirolimus is optionally modified. Examples of the sirolimus derivative may include ester, ether, amide, carbonate, carbamate, sulfonate, oxime, hydrazone, and hydroxyamine derivatives.

As the sirolimus, commercially available sirolimus may be used, sirolimus generated from *Streptomyces hygroscopicus*, which is a bacterium, by, for example, a method known to those skilled in the art may be used, or chemically synthesized sirolimus may be used.

The skin external medicine of the present embodiment is used for treating and/or preventing skin hardening in systemic sclerosis. The degree, stage, and the like of skin hardening in systemic sclerosis are not particularly limited. For example, the skin external medicine of the present embodiment can be used in early-stage skin fibrosis, and can also be used after progression of skin fibrosis.

In the present embodiment, the treatment may refer to complete disappearance of skin hardening, or may refer to amelioration of skin hardening after administration of the skin external medicine of the present embodiment even in a case in which skin hardening does not completely disappear. For example, after administration of the skin external medicine of the present embodiment, a reduction in dermal thickness in a lesion site, a decrease in the amount of soluble collagen in the skin tissue, suppression of inflammation in the skin tissue, a decrease in the expression level of phosphorylated mTOR in the skin tissue, or the like in comparison with that before the administration of the skin external medicine can be considered to show the amelioration of skin hardening. For example, when a dermal thickness in a lesion site, the amount of soluble collagen in the skin tissue, a value indicating the degree of inflammation (for example, the number of CD3 positive cells, the number of CD68 positive cells, the expression level of TNF-α, the expression level of TGF-β, or the like), the expression level of phosphorylated mTOR, or the like after administration of the skin external medicine of the present embodiment is a low value in comparison with a pre-determined cutoff value, skin hardening can be considered to be ameliorated. A therapeutic effect for skin hardening can be determined based on at least one index of such indices.

In the present embodiment, the prevention may refer to complete suppression of skin hardening from occurring from a condition in which skin hardening does not occur, or may refer to a reduction in the progression or severity of skin hardening. For example, a reduction in dermal thickness in a lesion site, a decrease in the amount of soluble collagen in the skin tissue, a decrease in the degree of inflammation in the skin tissue, a decrease in the expression level of phosphorylated mTOR in the skin tissue, or the like in a subject who has received the skin external medicine of the present embodiment in comparison with a subject who has not received the skin external medicine can be considered to show a reduction in the progression or severity of skin hardening. For example, when a dermal thickness in a lesion site, the amount of soluble collagen in the skin tissue, a value indicating the degree of inflammation (for example, the number of CD3 positive cells, the number of CD68 positive cells, the expression level of TNF-α, the expression level of TGF-β, or the like), the expression level of phosphorylated mTOR, or the like after administration of the skin external medicine of the present embodiment is a low value in comparison with a pre-determined cutoff value, the progression or severity of skin hardening can be considered to be reduced. A preventive effect for skin hardening can be determined based on at least one index of such indices.

A dermal thickness can be measured by a method known to those skilled in the art. The dermal thickness can be measured by, for example, staining the skin tissue, collected from a subject, with hematoxylin and eosin (HE), and microscopically observing the skin tissue.

The amount of soluble collagen in the skin tissue can be measured by a method known to those skilled in the art. The amount can be measured by, for example, performing Sircol assay using the skin tissue collected from a subject.

A value indicating the degree of inflammation in the skin tissue can be measured by a method known to those skilled in the art. The value showing the degree of inflammation can be measured by, for example, immunostaining the skin tissue collected from a subject, and microscopically observing the skin tissue to measure the number of cells related to inflammation, such as the number of CD3 positive cells or the number of CD68 positive cells. Moreover, a value indicating the degree of inflammation can be measured by measuring the expression level of a cytokine or the like related to inflammation, such as the expression level of TNF-α or the expression level of TGF-β, by carrying out a method such as a real-time PCR method using the skin tissue collected from a subject.

The expression level of phosphorylated mTOR in the skin tissue can be measured by a method known to those skilled in the art. The expression level can be measured by, for example, carrying out a method such as a Western blot method using the skin tissue collected from a subject.

The form of the skin external medicine of the present embodiment is not particularly limited, and may be a gel, a cream, an ointment, a liquid, a lotion, or a patch. Such forms can be selected depending on an application as appropriate. Even in the case of any form of the skin external medicine, the skin external medicine enables treatment with a few systemic side effects because the skin external medicine can exert a pharmacological effect only in a local site to which the skin external medicine is administered, unlike an oral medicine, due to the external medicine.

As long as the skin external medicine of the present embodiment includes sirolimus as an active ingredient, and the effects of the skin external medicine of the present embodiment are not adversely influenced, the other ingredients of the skin external medicine are not particularly limited, and can be selected depending on an application or the like as appropriate. For example, in a case in which the skin external medicine is a gel, the skin external medicine may include carboxyvinyl polymer, ethanol, 2,2',2"-nitrilotriethanol, or the like as a base material, and may further include another optional ingredient as necessary. Examples of such other optional ingredients may include pharmaceutically acceptable solvents and additives. Examples of the pharmaceutically acceptable solvents may include water, physiological saline, and buffer solutions. Examples of the additives may include stabilizers, pH adjusters, thickeners, antioxidants, isotonizing agents, buffer agents, solubilizing agents, suspending agents, preservatives, frost damage inhibitors, cryoprotective agents, freeze-drying protective agents, and bacteriostatics.

The skin external medicine of the present embodiment can be produced by a method known to those skilled in the art, and can be produced by a method including mixing the various ingredients described above. The concentrations of the various ingredients, temperature and time in the production, the pH of the skin external medicine, and the like can be prepared as appropriate, as necessary.

A commercially available skin external medicine may be used as the skin external medicine of the present embodiment. For example, in the case of using a gel, the gel may be produced by a method known to those skilled in the art. For example, RAPALIMUS (registered trademark) (product name) gel 0.2% (2 mg of sirolimus in 1 g) (Nobelpharma Co., Ltd.) can be used as a commercially available gel containing sirolimus.

The content of sirolimus in the skin external medicine of the present embodiment is not particularly limited unless the therapeutic effects of the skin external medicine of the present embodiment are adversely influenced. For example, the content may be 0.1% by mass or more, 0.2% by mass or more, 0.3% by mass or more, 0.4% by mass or more, 0.5% by mass or more, 1.0% by mass or more, 2.0% by mass or more, 5.0% by mass or more, or 10.0% by mass or more with respect to the total of the skin external medicine. Moreover, the content may be 30.0% by mass or less, 20.0% by mass or less, 15.0% by mass or less, 10.0% by mass or less, 5.0% by mass or less, 4.0% by mass or less, 3.0% by mass or less, 2.0% by mass or less, or 1.0% by mass or less with respect to the total of the skin external medicine. These upper and lower limits may be combined to form a certain range. For example, the content may be 0.1% by mass or more and 30.0% by mass or less, 0.2% by mass or more and 20.0% by mass or less, and 0.2% by mass or more and 5.0% by mass or less with respect to the total of the skin external medicine.

The dose of the skin external medicine of the present embodiment is not particularly limited unless the effects of the composition are adversely influenced. The dose can be adjusted as appropriate on the basis of the age, sex, body weight, degree or stage of skin hardening, or the like of a subject. For example, in the case of applying the skin external medicine containing such an amount of sirolimus as described above to the skin of a human adult (having a body weight of 60 kg), it is acceptable to apply 0.1 g to 10.0 g, 0.3 g to 5.0 g, 0.4 g to 2.0 g, or 0.4 g to 1.0 g of the skin external medicine per day. Specifically, for example, in the case of applying a gel of containing sirolimus in an amount of 0.2% by mass as a skin external medicine to the skin of a human adult (having a body weight of 60 kg), it is acceptable to apply 0.1 g to 10.0 g, 0.3 g to 5.0 g, 0.4 g to 2.0 g, or 0.4 g to 1.0 g of the skin external medicine per day.

The number of times of the application may be one per day, one to two per day, or one to three per day.

A site to which the skin external medicine of the present embodiment is applied is not particularly limited as long as the site is a site in which skin hardening in systemic sclerosis occurs or a site in which skin hardening is prone to occur. Examples of such sites include the skins of the fingers, the skins of the forearms, and the skin of the face.

A subject animal to which the skin external medicine of the present embodiment is applied is not particularly limited as long as the animal is a mammalian animal in which skin hardening in systemic sclerosis occurs. Examples of such subject animals include humans and mice.

The skin external medicine of the present embodiment may be a skin external medicine that suppresses production of collagen in the skin to which the skin external medicine is administered.

The suppression of production of collagen may refer to complete disappearance of collagen produced in the lesion site of the skin hardening of a subject, or may refer to a decrease the amount of soluble collagen after administration of the skin external medicine of the present embodiment even in a case in which produced collagen does not completely disappear. For example, after administration of the skin external medicine of the present embodiment, a decrease in the amount of soluble collagen in the skin tissue in comparison with that before the administration of the skin external medicine, or the like can be considered to show the suppression of production of collagen. For example, when the amount of soluble collagen in the skin tissue after administration of the skin external medicine of the present embodiment is a low value in comparison with a pre-determined cutoff value, production of collagen can be considered to be suppressed.

Alternatively, the suppression of production of collagen may refer to complete suppression of production of collagen from a condition in which collagen is not produced in the skin of a subject, particularly in the skin tissue in which skin hardening can occur, and may refer to suppression of an increase in production of collagen. For example, a decrease in the amount of soluble collagen in the skin tissue in a subject who has received the skin external medicine of the present embodiment in comparison with a subject who has not received the skin external medicine can be considered to show suppression of production of collagen. For example, when the amount of soluble collagen in the skin tissue, or the like after administration of the skin external medicine of the present embodiment is a low value in comparison with a pre-determined cutoff value, production of collagen can be considered to be suppressed.

The skin external medicine of the present embodiment may suppress inflammation in the skin to which the skin external medicine is administered.

The suppression of inflammation may refer to complete disappearance of inflammation in the lesion site of the skin hardening of a subject, or may refer to a decrease in inflammation after administration of the skin external medicine of the present embodiment even in a case in which inflammation does not completely disappear. For example, after administration of the skin external medicine of the present embodiment, a decrease in a value indicating the degree of inflammation in the skin tissue (for example, the number of CD3 positive cells, the number of CD68 positive cells, the expression level of TNF-α, the expression level of TGF-β, or the like), or the like in comparison with that before the administration of the skin external medicine can be considered to show suppression of inflammation. For example, when a value indicating the degree of inflammation in the skin tissue after administration of the skin external medicine of the present embodiment is a low value in comparison with a pre-determined cutoff value, inflammation can be considered to be suppressed.

Alternatively, the suppression of inflammation may refer to complete suppression of occurrence of inflammation from a condition in which inflammation does not occur in the skin of a subject, particularly in the skin tissue in which skin hardening can occur, and may refer to suppression of an increase in inflammation. For example, a decrease in a value indicating the degree of inflammation in the skin tissue (for example, the number of CD3 positive cells, the number of CD68 positive cells, the expression level of TNF-α, the expression level of TGF-β, or the like), or the like in a subject who has received the skin external medicine of the present embodiment in comparison with a subject who has not received the skin external medicine can be considered to show suppression of inflammation. For example, when a value indicating the degree of inflammation in the skin tissue after administration of the skin external medicine of the present embodiment is a low value in comparison with a pre-determined cutoff value, inflammation can be considered to be suppressed.

The skin external medicine of the present embodiment exerts the therapeutic or preventive effects described above on the lesion site of skin hardening of a subject with systemic sclerosis, preferably through suppression of an mTOR signal by sirolimus.

The suppression of an mTOR signal may refer to complete disappearance of an mTOR signal in the lesion site of the skin hardening of a subject, or may refer to a decrease in an mTOR signal after administration of the skin external medicine of the present embodiment even in a case in which an mTOR signal does not completely disappear. For example, after administration of the skin external medicine of the present embodiment, a decrease in the expression level of phosphorylated mTOR in the skin tissue, or the like in comparison with that before the administration of the skin external medicine can be considered to show suppression of an mTOR signal. For example, when the expression level of phosphorylated mTOR in the skin tissue after administration of the skin external medicine of the present embodiment is a low value in comparison with a pre-determined cutoff value, an mTOR signal can be considered to be suppressed.

Alternatively, the suppression of an mTOR signal may refer to complete suppression of occurrence of an mTOR signal from a condition in which an mTOR signal does not occur in the skin of a subject, particularly in the skin tissue in which skin hardening can occur, and may refer to suppression of an increase in mTOR signal. For example, a decrease in the expression level of phosphorylated mTOR in the skin tissue, or the like in a subject who has received the skin external medicine of the present embodiment in comparison with a subject who has not received the skin external medicine can be considered to show suppression of an mTOR signal. For example, when the expression level of phosphorylated mTOR in the skin tissue after administration of the skin external medicine of the present embodiment is a low value in comparison with a pre-determined cutoff value, an mTOR signal can be considered to be suppressed.

Other examples of the present embodiment may include the following:
a method of treating or preventing skin hardening in systemic sclerosis, wherein the method comprises administering sirolimus to the skin of a subject in need thereof;
sirolimus for use in treatment or prevention of skin hardening in systemic sclerosis by administration of the sirolimus to the skin; and
use of sirolimus in manufacturing of a skin external medicine for treating or preventing skin hardening in systemic sclerosis.

### Examples

The present invention is described in more detail below with reference to Examples. However, it will be appreciated that the scope of the present invention is not limited only to Examples.

### <Example 1: Suppressive Effect of Sirolimus Gel on Skin Fibrosis in Systemic Sclerosis>

It was examined whether skin fibrosis was suppressed by external use of sirolimus gel (Nobelpharma Co., Ltd., product name: RAPALIMUS (registered trademark) Gel 0.2%: 2 mg of sirolimus in 1 g) on the skin with early-stage fibrosis. Scleroderma-like skin hardening model mice were produced by subcutaneously injecting 300 µg of bleomycin (Nippon Kayaku Co., Ltd.) into C57BL/6 mice (Japan SLC, Inc., 8-week-old, female) per day. Mice into which PBS was subcutaneously injected instead of bleomycin were used as control mice. Hereinafter, the first day on which bleomycin was administered was regarded as day 1.

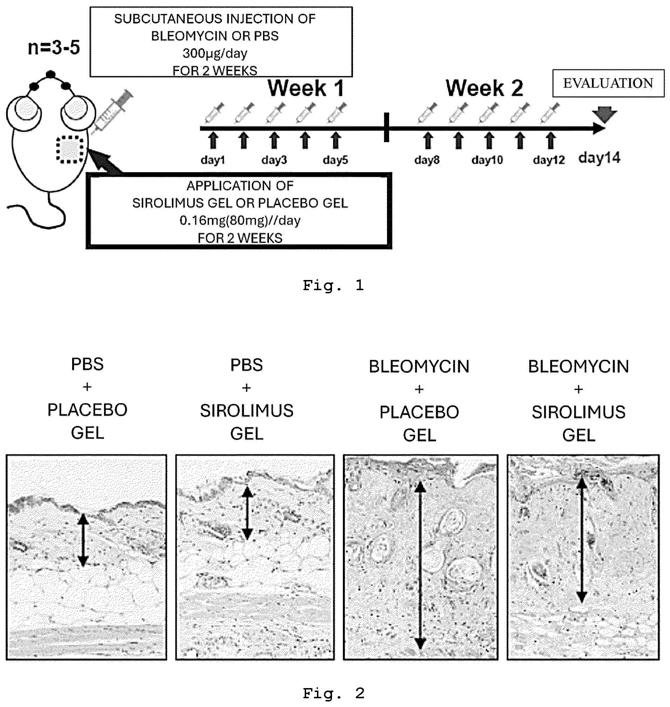

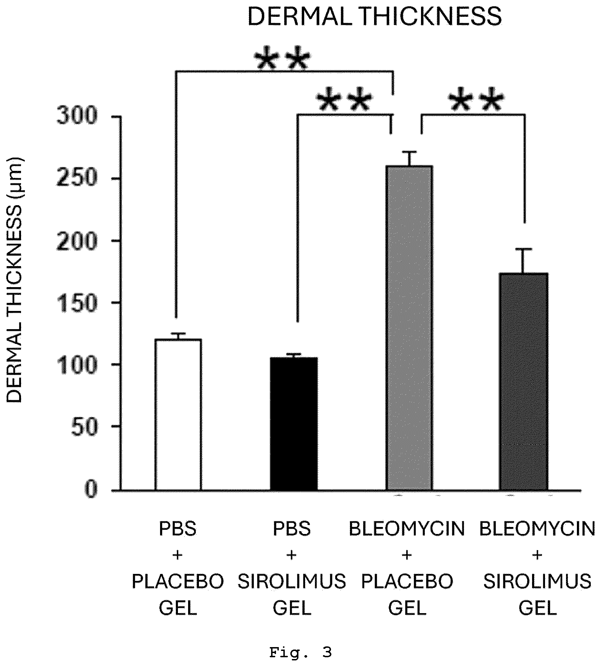

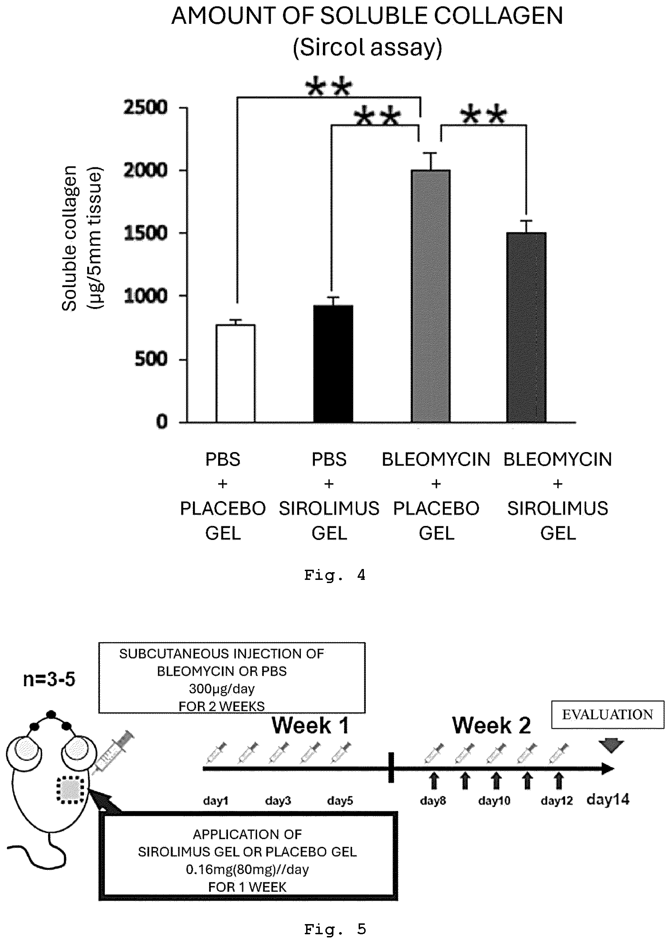

In Example 1, external use of sirolimus gel was also started from day 1 according to a dosing schedule illustrated in Figure 1 in order to confirm the effect of sirolimus on early-stage skin fibrosis. The sirolimus gel was applied to the skin so that the amount of sirolimus was 0.16 mg (the amount of the gel was 80 mg) per day. The sirolimus-containing gel and a gel including no sirolimus (a gel including a base having exactly the same composition as the composition of the base of the sirolimus-containing gel, and differing from the sirolimus-containing gel only in including no sirolimus; that is, a placebo gel; provided from Nobelpharma Co., Ltd.) as a control were used externally. The results of collecting the skin tissue from each model mouse 14 days (day 14) after the start of the administration of bleomycin and performing HE staining of the skin tissue by a method known to those skilled in the art are illustrated in Figures 2 to 3. Figure 2 illustrates representative examples of the results of performing microscopic observation, and Figure 3 illustrates a graph of a dermal thickness (one-way ANOVA and Tukey's test; n = 3 to 5; in the figure, ** denotes p < 0.01, and each error bar denotes mean + SEM (standard error)). As a result, it was able to be confirmed that the dermal thickness of each scleroderma-like skin hardening model mouse on which the placebo gel was used externally was increased while the dermal thickness of each scleroderma-like skin hardening model mouse on which the sirolimus gel was used externally was small in comparison with the case of the external use of the placebo gel. Furthermore, the amount of soluble collagen in the skin tissue of the lesion of each mouse was measured by using specific binding of collagen and Sirius red by Sircol assay (Sircol Soluble Collagen Assay Kit, biocolor) as a method known to those skilled in the art, as illustrated in Figure 4. As a result, it was revealed that the content of collagen in each scleroderma-like skin hardening model mouse on which the sirolimus gel was used externally was suppressed in comparison with the case of the external use of the placebo gel (one-way ANOVA and Tukey's test; n = 3 to 5; in the figure, ** denotes p < 0.01, and each error bar denotes mean + SEM (standard error)). Based on the above, it was revealed that skin fibrosis is suppressed by externally using sirolimus gel from the early stage of skin fibrosis.

The mean of the mean body weights of the C57BL/6 mice (8-week-old, female) used in Examples in the present disclosure, respectively, was 20.1 g. An effect such as suppression of skin fibrosis was able to be confirmed in the case of applying the gel to the skin of each of the mice so that the amount of sirolimus was 0.16 mg (the amount of the gel was 80 mg) per day. When this dose is converted into a dose for a human having a body weight of 60 kg, the dose of the gel is about 0.48 g. The same applies to the following Examples.

### <Example 2: Therapeutic Effect of Sirolimus Gel on Skin Fibrosis in Systemic Sclerosis>

It was examined whether skin fibrosis was able to be treated by external use of sirolimus gel on the fibrotic skin. Scleroderma-like skin hardening model mice were produced by subcutaneously injecting 300 µg of bleomycin into C57BL/6 mice (Japan SLC, Inc., 8-week-old, female) per day. Mice into which PBS was subcutaneously injected instead of bleomycin were used as control mice. Hereinafter, the first day on which bleomycin was administered was regarded as day 1.

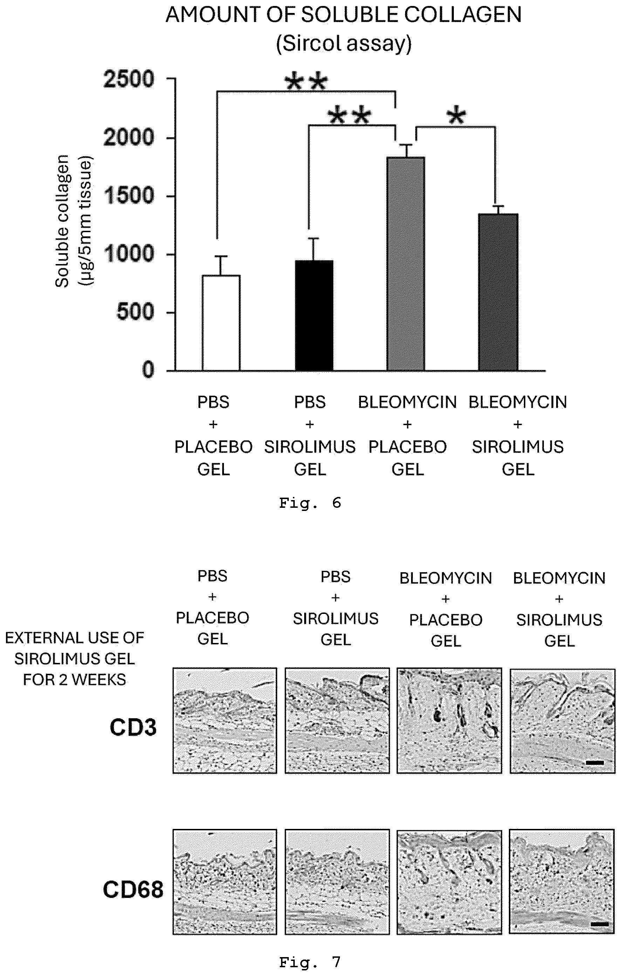

In Example 2, external use of sirolimus gel (Nobelpharma Co., Ltd., product name: RAPALIMUS (registered trademark) Gel 0.2%: 2 mg of sirolimus in 1 g) was also started from day 8 according to a dosing schedule illustrated in Figure 5 in order to confirm the effect of sirolimus on the fibrotic skin. The sirolimus gel was applied to the skin so that the amount of sirolimus was 0.16 mg (the amount of the gel was 80 mg) per day. The sirolimus-containing gel and a gel including no sirolimus (a gel including a base having exactly the same composition as the composition of the base of the sirolimus-containing gel, and differing from the sirolimus-containing gel only in including no sirolimus; that is, a placebo gel; provided from Nobelpharma Co., Ltd.) as a control were used externally. The skin tissue was collected from each model mouse 14 days (day 14) after the start of the administration of bleomycin. The amount of soluble collagen in the skin tissue of the lesion of each mouse was measured by using specific binding of collagen and Sirius red by Sircol assay as a method known to those skilled in the art, as illustrated in Figure 6. As a result, it was revealed that the content of collagen in each scleroderma-like skin hardening model mouse on which the sirolimus gel was used externally was decreased in comparison with the case of the external use of the placebo gel (one-way ANOVA and Tukey's test; n = 3 to 5; in the figure, ** denotes p < 0.01, and each error bar denotes mean + SEM (standard error)). Based on the above, it was revealed that skin fibrosis can be treated by externally using sirolimus gel on the fibrotic skin.

### <Example 3: Effect of Sirolimus Gel on Inflammation of Skin Fibrosis Site in Systemic Sclerosis>

The serum levels of various cytokines, particularly Th2 cytokine, Th17 cytokine, and TGF-β, are known to increase in a patient with systemic sclerosis (Non Patent Literature 3 and 4). Moreover, abnormal activation of immune cells such as T cells, B cells, NK cells, and macrophages is confirmed in systemic sclerosis (Non Patent Literature 4 and 5). Moreover, immune activation is known to lead to fibrosis through the stimulation of production of collagen by fibroblasts (Non Patent Literature 6). In other words, it is suggested that immunologic abnormality is involved in the medical condition of systemic sclerosis.

It was examined whether an effect on inflammation of a skin fibrosis site was also obtained in the case of external use of sirolimus. Scleroderma-like skin hardening model mice were produced in a manner similar to that in Example 1. In other words, the scleroderma-like skin hardening model mice were produced by subcutaneously injecting 300 µg of bleomycin into C57BL/6 mice (Japan SLC, Inc., 8-week-old, female) per day. Mice into which PBS was subcutaneously injected instead of bleomycin were used as control mice. Hereinafter, the first day on which bleomycin was administered was regarded as day 1.

In Example 3, external use of sirolimus gel (Nobelpharma Co., Ltd., product name: RAPALIMUS (registered trademark) Gel 0.2%: 2 mg of sirolimus in 1 g) was also started from day 1 according to a dosing schedule illustrated in Figure 1. The sirolimus gel was applied to the skin so that the amount of sirolimus was 0.16 mg (the amount of the gel was 80 mg) per day. The sirolimus-containing gel and a gel including no sirolimus (a gel including a base having exactly the same composition as the composition of the base of the sirolimus-containing gel, and differing from the sirolimus-containing gel only in including no sirolimus; that is, a placebo gel; provided from Nobelpharma Co., Ltd.) as a control were used externally.

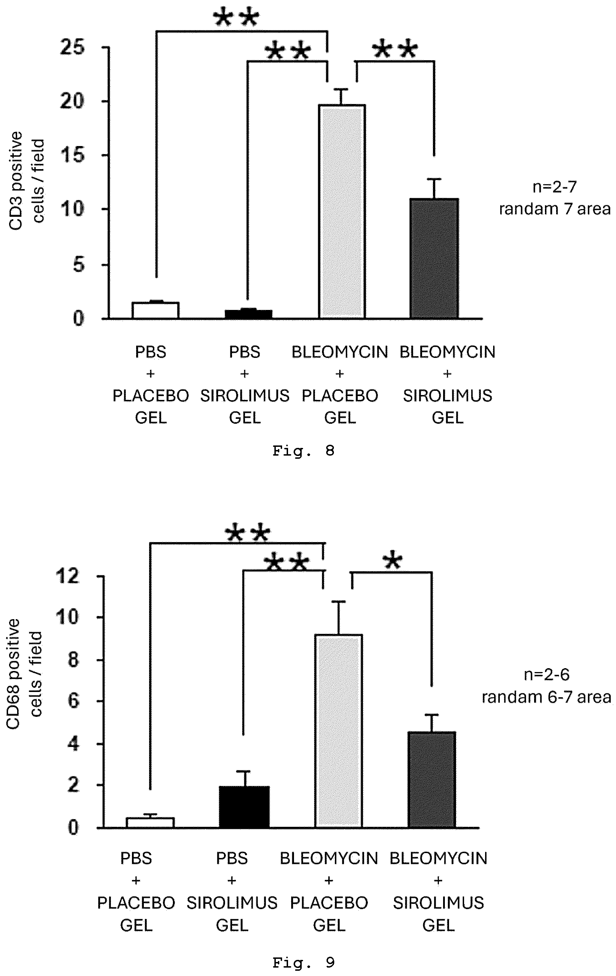

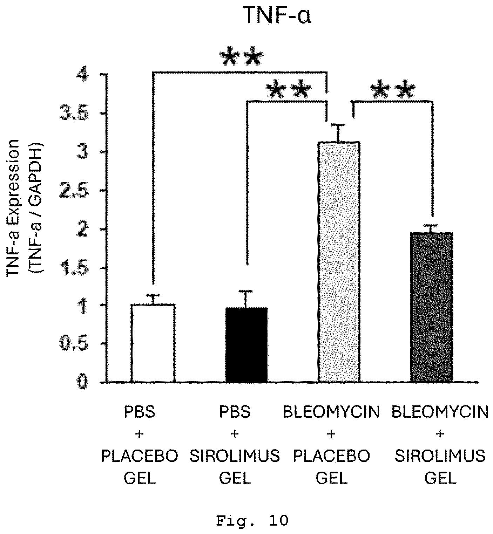

The results of collecting the skin tissue from each model mouse 14 days (day 14) after the start of the administration of bleomycin and immunostaining the skin tissue with an anti-CD3 antibody (Abcam) and an anti-CD68 antibody (Bio-Rad) by a method known to those skilled in the art are illustrated in Figures 7 to 9. Figure 7 illustrates representative examples of the results of performing microscopic observation, and Figures 8 to 9 illustrate graphs of the numbers of CD3 positive cells and CD68 positive cells (one-way ANOVA and Tukey's test; n = 2 to 7; random measurement in six to seven areas in each mouse; in the figures, ** denotes p < 0.01, * denotes p < 0.05, and each error bar denotes mean + SEM (standard error)). As a result, it was able to be confirmed that the numbers of the CD3 positive cells and the CD68 positive cells were significantly increased in each scleroderma-like skin hardening model mouse on which the placebo gel was used externally while increases in the numbers of CD3 positive cells and CD68 positive cells were significantly suppressed in each scleroderma-like skin hardening model mouse on which the sirolimus gel was externally used in comparison with the case of the external use of the placebo gel. Furthermore, the expression levels of TNF-α and TGF-β in the skin of the lesion of each mouse were measured by a real-time PCR method as a method known to those skilled in the art, as illustrated in Figures 10 to 11. For primers used in the real-time PCR method, TNFα mouse TNF-α Forward (sigma): 5'-GCCTCTTCTCATTCCTGCTTG-3' (SEQ ID NO:1) was used as a forward primer, and TNFα mouse TNF-α Reverse (sigma): 5'-CTGATGAGAGGGAGGCCATT-3' (SEQ ID NO:2) was used as a reverse primer in the measurement of the expression level of TNF-α. For the measurement of the expression level of TGF-β, MA148599-F (Tgfb1) (takara): 5'-TACGGCAGTGGCTGAACCAA-3' (SEQ ID NO:3) was used as a forward primer, and MA148599-R (Tgtb1) (takara): 5'-CGGTTCATGTCATGGATGGTG-3' (SEQ ID NO:4) was used as a reverse primer. As a result, it was revealed that the expression level of TNF-α was significantly decreased, and the expression level of TGF-β was slightly decreased while no significant difference of the expression level of TGF-β was shown, in each scleroderma-like skin hardening model mouse on which the sirolimus gel was externally used, in comparison with the case of the external use of the placebo gel (one-way ANOVA and Tukey's test; n = 2 to 7; in the figures, ** denotes p < 0.01, and each error bar denotes mean + SEM (standard error)). Based on the above, it was revealed that external use of sirolimus gel on a skin fibrosis site in systemic sclerosis allows suppression of infiltration of inflammatory cells generated in the site (that is, CD3 positive T cells and CD68 positive macrophages), and also allows reductions in the amounts of inflammatory cytokines such as TNF-α.

### <Example 4: Influence of Sirolimus Gel on Expression of Phosphorylated mTOR in Skin Fibrosis Site in Systemic Sclerosis>

It is confirmed that sirolimus as an mTOR inhibitor inhibits a signal transfer mechanism dependent on IL-2 and other cytokine receptors by inhibiting the activation of both T cells and B cells (Non Patent Literature 7), and fibrosis-induced inflammatory cytokines (IL-4, IL-61, IL-17, and TGF-β1) were suppressed by intraperitoneal administration of sirolimus (Non Patent Literature 2). In other words, it is suggested that sirolimus has an immunosuppressive action.

It was examined whether an mTOR inhibitory effect was also obtained in the case of external use of sirolimus. Scleroderma-like skin hardening model mice were produced in a manner similar to that in Example 1. In other words, the scleroderma-like skin hardening model mice were produced by subcutaneously injecting 300 µg of bleomycin into C57BL/6 mice (Japan SLC, Inc., 8-week-old, female) per day. Mice into which PBS was subcutaneously injected instead of bleomycin were used as control mice. Hereinafter, the first day on which bleomycin was administered was regarded as day 1.

In Example 4, external use of sirolimus gel (Nobelpharma Co., Ltd., product name: RAPALIMUS (registered trademark) Gel 0.2%: 2 mg of sirolimus in 1 g) was also started from day 1 according to a dosing schedule illustrated in Figure 1. The sirolimus gel was applied to the skin so that the amount of sirolimus was 0.16 mg (the amount of the gel was 80 mg) per day. The sirolimus-containing gel and a gel including no sirolimus (a gel including a base having exactly the same composition as the composition of the base of the sirolimus-containing gel, and differing from the sirolimus-containing gel only in including no sirolimus; that is, a placebo gel) as a control were used externally.

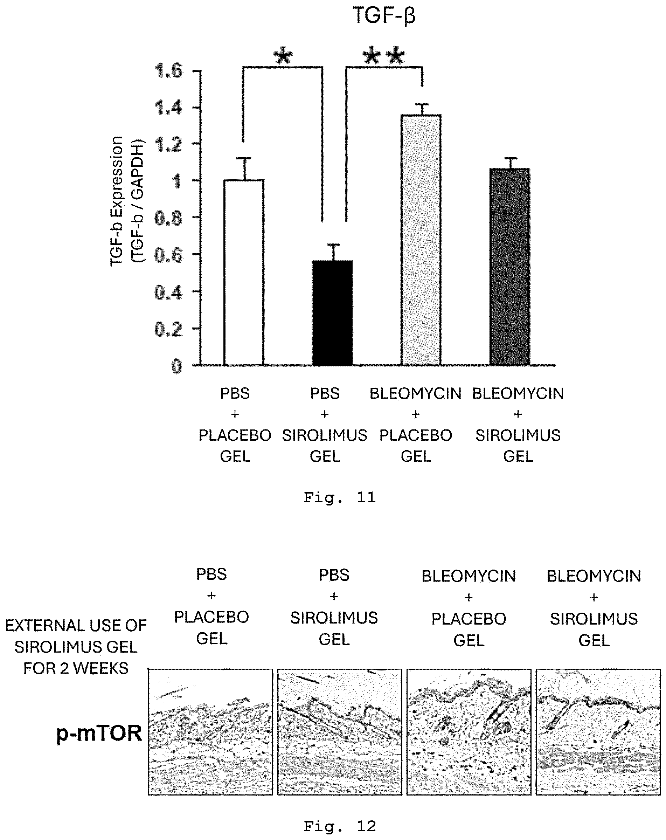

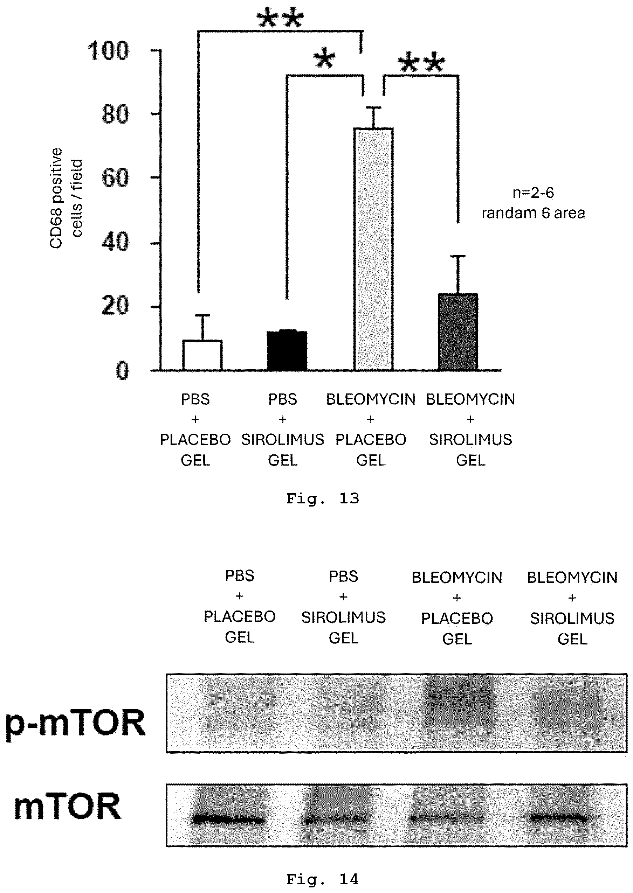

The results of collecting the skin tissue from each model mouse 14 days (day 14) after the start of the administration of bleomycin and immunostaining the skin tissue with an anti-p-mTOR antibody (Cell Signaling) by a method known to those skilled in the art are illustrated in Figures 12 to 14. Figure 12 illustrates representative examples of the results of performing microscopic observation, and Figure 13 illustrates a graph of the number of phosphorylated mTOR (p-mTOR) expressed cells (one-way ANOVA and Tukey's test; n = 2 to 6; random measurement in six areas in each mouse; in the figure, ** denotes p < 0.01, * denotes p < 0.05, and each error bar denotes mean + SEM (standard error)). As a result, it was able to be confirmed that the number of p-mTOR expressed cells was significantly increased in each scleroderma-like skin hardening model mouse on which the placebo gel was used externally while an increase in the number of p-mTOR expressed cells was significantly suppressed in each scleroderma-like skin hardening model mouse on which the sirolimus gel was externally used in comparison with the case of the external use of the placebo gel. Furthermore, the expression of p-mTOR in the skin of the lesion of each mouse was confirmed by a Western blot method as a method known to those skilled in the art, as illustrated in Figure 14. Phospho-mTOR (Ser2448) Antibody #2971 (Cell Signaling) was used as a primary antibody, and Anti-rabbit IgG, HRP-linked Antibody #7074 (Cell Signaling) was used as a secondary antibody. As a result, it was revealed that the expression level of p-mTOR was reduced in each scleroderma-like skin hardening model mouse on which the sirolimus gel was externally used, in comparison with the case of the external use of the placebo gel. Based on the above, it was revealed that external use of sirolimus gel on a skin fibrosis site in systemic sclerosis allows suppression of elevated expression of p-mTOR in the site.

These results suggested that a therapeutic effect is obtained by external use of a sirolimus-containing gel on skin hardening in a patient with systemic sclerosis. It is expected that the present invention can provide a new treatment method with a few side effects and high safety, against skin hardening in sclerosis, against which no effective and safe treatment method exists.

## Claims

1. A skin external medicine for treatment or prevention of skin hardening in systemic sclerosis, wherein the skin external medicine comprises sirolimus.

2. The skin external medicine according to claim 1, wherein the treatment or the prevention results in suppression of early-stage skin fibrosis.

3. The skin external medicine according to claim 1 or 2, wherein a dosage form is a gel, a cream, an ointment, a liquid, a lotion, or a patch.

4. The skin external medicine according to claim 1 or 2, wherein the skin external medicine comprises sirolimus at a rate of 0.2% by mass or more with respect to a total of the skin external medicine.

5. The skin external medicine according to claim 1 or 2, wherein production of collagen is suppressed in a skin to which the skin external medicine is administered.

6. The skin external medicine according to claim 1 or 2, wherein inflammation is suppressed in a skin to which the skin external medicine is administered.
